# EUROPEAN PATENT SPECIFICATION

(11) **EP 2 018 204 B1**
(45) Date of publication and mention of the grant of the patent: **27.06.2012**
(21) Application number: 07783227.7
(22) Date of filing: 03.05.2007
(51) Int. Cl.: A61M 25/01, A61M 25/00

(54) **STEERABLE CATHETER USING FLAT PULL WIRES AND METHOD OF MAKING SAME**
LENKBARER KATHETER MIT FLACHEN ZUGDRÄHTEN UND HERSTELLUNGSVERFAHREN DAFÜR
CATHÉTER ORIENTABLE AU MOYEN DE FILS DE TRACTION PLATS ET PROCÉDÉ DE FABRICATION DE CELUI-CI

(30) Priority: 16.05.2006 US 800373 P; 29.12.2006 US 647313
(43) Date of publication of application: 28.01.2009
(73) Proprietor: St. Jude Medical, Atrial Fibrillation Division, Inc., St. Paul, Minnesota 55117-9913 (US)
(72) Inventor: CUMMING, Sarah, Plymouth, MN 55442 (US); DUSTRUDE, Mark, Minnetonka, MN 55345 (US); FUENTES, Allan M., Mound, MN 55346 (US); HEIDEMAN, Wayne, Minnetonka, MN 55345 (US); STEHR, Richard E., Stillwater, MN 55082 (US)
(74) Representative: Allgayer, Ulla Barbara
(86) International application number: PCT/US2007/068176
(87) International publication number: WO 2007/136981

(56) References cited:
- WO-A1-99/33509
- WO-A2-01/78825
- US-A- 4 306 029
- US-A- 5 238 005
- US-A- 5 487 757
- US-A1- 2002 077 590
- US-A1- 2002 177 772
- US-A1- 2005 038 467
- US-A1- 2005 107 737
- US-B2- 6 582 536

## Description

### CROSS-REFERENCE TO RELATED APPLICATIONS

This application claims the benefit of United States provisional application no. 60/800,373 filed on 16 May 2006 (the '373 application). This application also claims the benefit of United States application no. 11/647,313, filed 29 December 2006 (the '313 application), now pending.

### BACKGROUND OF THE INVENTION

### a. Field of the Invention

The present invention pertains generally to catheters that are used in the human body. More particularly, the present invention is directed to steerable catheters using flat pull wires to reduce the overall outer dimension of the catheter.

### b. Background Art

Catheters are used for an ever-growing number of procedures. For example, catheters are used for diagnostic, therapeutic, and ablative procedures, to name just a few examples. Typically, the catheter is manipulated through the patient's vasculature and to the intended site, for example, a site within the patient's heart. The catheter typically carries one or more electrodes, which may be used for ablation, diagnosis, or the like.

Many prior catheters use round wires as pull wires, and they typically either embed the wire directly into the catheter wall so that the pull wire and the lumen through which it runs are substantially the same size, or use a round wire to create a pull wire lumen and then place a smaller wire in the lumen as a pull wire. These conventional techniques and methods result in a catheter that is elliptical in its outer shape. An example of an elliptical catheter is disclosed and taught in United States Patent No. 6,582,536, the contents of which are incorporated herein by reference.

As catheters are used in smaller and smaller passages, there is a growing need to use catheters that have a smaller outer dimension. Accordingly, there is a need to use steerable catheters that have smaller cross-sections.
WO 99/33509 discloses a deflectable guiding catheter generally having an elongated shaft with a deflectable distal section, a second lumen, a first lumen in fluid communication with a port in a distal end of the shaft, an elongated tapered deflection line within the second lumen and reinforcement strands within a wall of the shaft.

US 6,582,536 B2 discloses a method for manufacturing a steerable catheter having a distal end, a proximal end, an outer jacket, a pull wire and a central lumen.

### BRIEF SUMMARY OF THE INVENTION

According to a first embodiment of the invention, a catheter assembly includes an inner liner made of flexible material and an outer layer having a steering mechanism. The steering mechanism includes at least one flat wire and a corresponding lumen for each of the at least one flat wire through which the flat wire may travel. Optionally, the catheter assembly may include a layer of heat shrink material encompassing the outer layer, a central lumen, and/or a braided wire assembly contained in the outer layer. The overall cross-section of the catheter assembly may be substantially circular. The outer layer typically comprises a melt processing polymer such that the catheter assembly may be laminated using heat.

Optionally, the flat wire or wires may be encased in a preformed tube in which the flat wire may travel. The flat wire may have a rectangular cross-section, typically having dimensions of about X by about 3X, and the cross-section of the preformed tube may be oval, round, or elliptical. That is, the cross-section of the preformed tube may be of a different shape than the cross-section of the flat wire disposed therein. The flat wire may be coated with a lubricious substance to permit the flat wire to slide in its lumen, or optionally, the flat wire may be manufactured with a smooth surface to reduce friction between the flat wire and its lumen.

The braided wire assembly may extend from a base of the catheter assembly to a distal end of the catheter assembly, and a braid density may transition from a first braid density at the base to a lower braid density at the distal end. For example, the braid density may be about 50 PPI at the base and about 10 PPI at the distal end. Alternatively, the braid density at the distal end may be about 20% to about 35% of the braid density at the base.

Also disclosed is a method of manufacturing a catheter including the steps of: providing a mandrel; placing a lining material over the mandrel to form an inner liner; providing at least one flat shaped wire; placing a flexible liner over each of the at least one flat shaped wires to create at least one flat lumen; placing a braided wire assembly over the inner liner and the at least one flat lumen; covering the braided wire assembly with a melt processing polymer; applying sufficient heat to the melt processing polymer to raise the temperature of the polymer above its melting point; cooling the assembly; and removing the mandrel, thereby forming a catheter. Typically, the catheter is manufactured such that it has a cross-section with an outer shape that is substantially circular with an outer diameter of less than about 4mm 12F. Optionally, the melt processing polymer may be covered with shrink wrap tubing to help promote the polymer flowing through the braided wire assembly. The shrink wrap tubing may be left in place after manufacturing, or it may be removed as part of the manufacturing process. The melt processing polymer is typically selected from Nylon, Pebax and other thermal elastomers. Optionally, additional layers of melt processing polymers may be placed over the flat lumen and the inner liner. Typically, the flat wire and the flexible liner being placed over the flat wire will each have different cross-sectional shapes.

Also disclosed is a method of manufacturing a steerable introducer catheter, including the steps of: providing a mandrel; laminating the mandrel with a lining material to form an inner liner; providing at least one flat shaped wire; covering the inner liner and the at least one flat shaped wire with a melt processing polymer; applying sufficient heat to the melt processing polymer to raise the temperature of the polymer above its melting point; cooling the assembly; and removing the mandrel, thereby forming a steerable introducer catheter. Optionally, a flexible tube is placed over each of the at least one flat shaped wires to create at least one corresponding lumen for each of the wires, and further, the melt processing polymer may be covered with a layer of shrink wrap tubing. The braided wire assembly may be characterized by a braid density that transitions from a first number at the base to a lower number at the tip. The variation in braid density may range from about 50 PPI at the base to about 10 PPI at the distal end.

The catheter assembly of the present invention may also include a pull ring to which the at least two flat wires are secured. The pull ring may be a right circular cylinder having a slot for each of the at least two flat wires. Typically, there are two flat wires, the pull ring has two slots spaced on opposite sides of the pull ring, and each of the flat wires is secured in the slot by a laser weld. The pull ring may further include at least two flow holes such that the outer layer will bond to the pull ring during melt processing as the melt processing polymer flows through the flow holes and then becomes rigid after cooling.

The catheter assembly of the present invention may also include a shaft made of at least three segments, wherein each segment has a different hardness characteristic. For example, a first shaft segment may be made of nylon, a second segment may be made of a first Pebax, and a third segment may be made of a second Pebax that is more flexible than both the nylon and the first Pebax. Additional segments may be used to form the shaft, each of which may have a greater or lesser degrees of stiffness.

Also disclosed is a pull ring assembly for a catheter including a pull ring having at least one rectangular slot and at least one flat pull wire, wherein each of the at least one flat pull wires is secured to the at least one rectangular slot of the pull ring. Typically, the pull ring assembly will include at least two slots and at least two flat pull wires secured in the slots. Optionally, the pull ring may include flow holes though which a melt processing polymer may flow during lamination.

According to another aspect a pull ring assembly includes a pull ring having at least two rectangular slots and at least two pull wires, wherein each of the at least two pull wires is secured to the rectangular slot of the pull ring. Optionally, the pull ring may include flow holes though which a melt processing polymer may flow during lamination.

A technical advantage of the present invention is that overall cross-section of the catheter may be reduced.

Another technical advantage of the present invention is that a steerable catheter using flat pull wires may be provided that enjoys greater flexibility.

Yet another technical advantage of the invention is it may utilize an improved braided wire assembly that provides for greater flexibility and control of a catheter.

A further technical advantage of the invention is that a method of manufacturing an improved steerable catheter is provided.

Yet another technical advantage of the invention is that a catheter shaft having greater flexibility and control may be utilized.

A further technical advantage of the invention is that a method of manufacturing an introducer with a lower profile outer diameter with improved steerability is provided.

The foregoing and other aspects, features, details, utilities, and advantages of the present invention will be apparent from reading the following description and claims, and from reviewing the accompanying drawings.

### BRIEF DESCRIPTION OF THE DRAWINGS

Figure 1 is perspective view of an embodiment of a catheter of the present invention.

Figure 2 illustrates a perspective view of a section of a catheter according to an embodiment of the present invention, cut away to show details.

Figure 3 is a cross-sectional view taken along line 3-3 in Figure 2.

Figure 4 is a cross-sectional view taken along line 4-4 in Figure 2.

Figure 5 is a cross-sectional view taken along line 5-5 in Figure 2.

Figure 6 is a cross-sectional view of a catheter assembly prior to the application of heat to melt process the outer layer.

Figure 7 is a cross-sectional view of a catheter after the application of heat to melt process the outer layer.

Figure 8 illustrates a perspective view of a partially assembled catheter in accordance with another embodiment of the invention, cut away to show details.

Figure 9 illustrates a pull ring that may be used in a catheter according to the present invention.

Figure 10 is a sectional view of the pull ring of Figure 9 taken along line 10-10.

### DETAILED DESCRIPTION OF THE INVENTION

The present invention provides an improved steerable catheter that minimizes the overall outer dimensions by utilizing a variety of improved techniques. One technique is to utilize flat wire as the pull wires for the steerable catheter.

For purposes of this invention, a "flat wire" or a "flat pull wire" refers to a wire that is characterized by a cross-section that, when measured along two orthogonal axes, is substantially flat. A flat wire typically has a rectangular cross-section For example, the rectangular cross-section may be approximately 0,1016 × 0,3048 mm 0.004" x 0.012". The cross-section need not be perfectly rectangular. For example, the present invention contemplates a cross-section of the flat wire may be oval, provided that the overall cross-section is generally flat. For example, a wire may be properly characterized as a flat wire if it has a cross-section that is measured X in one direction and at least 3X in a second direction generally orthogonal to the first direction. A wire whose cross-section is substantially I-shaped may also be a flat wire if, generally, its height is substantially greater than its width at its widest measurement. One of ordinary skill will appreciate that a flat wire may be defined in the context of the overall teachings of this application.

The use of a flat wire as a pull wire also has the added benefit that it provides greater resistance to deflection in certain directions. The shape of a round wire is not predisposed to resist deflection in any particular direction, whereas the shape of a flat wire will be predisposed to resist deflection on a first axis, and yet predisposed to permit deflection on a second axis that is orthogonal to the first axis. Thus, by using a pull wire that is not circular, a catheter can be predisposed to permit and favor deflection in one direction over another.

The outer diameter of the catheter may also be minimized at the distal tip by an improved braided wire assembly. In particular, a braid may be used that is characterized by a varying braid density from the proximal end to the distal tip. Preferably, the braid is less dense at the tip than at the proximal end of the catheter. Some applications may be better suited if the braid density is more dense at the tip than at the proximal end, while other applications may be better suited if the braid density is greater on both ends than in the middle of the catheter.

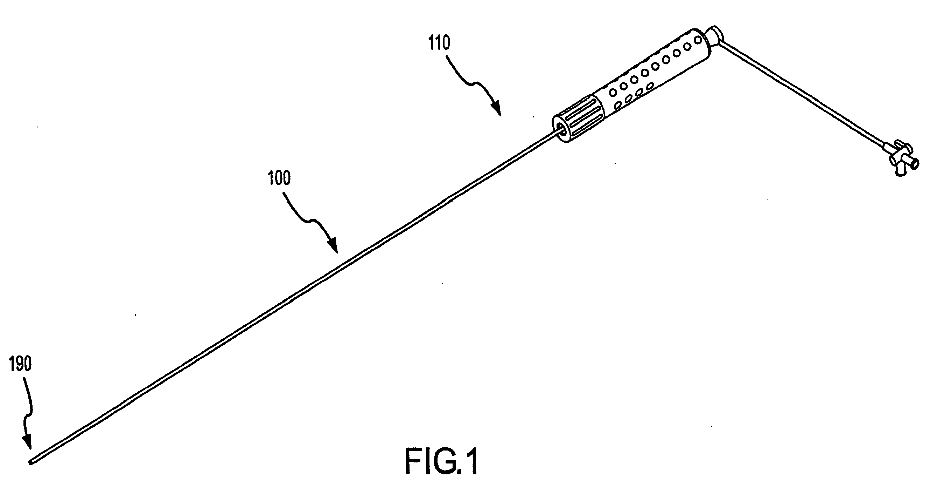

Figure 1 is a perspective view of a preferred embodiment of a catheter 100 of the present invention. Catheter 100 has a proximal portion 110 and a distal portion 190.

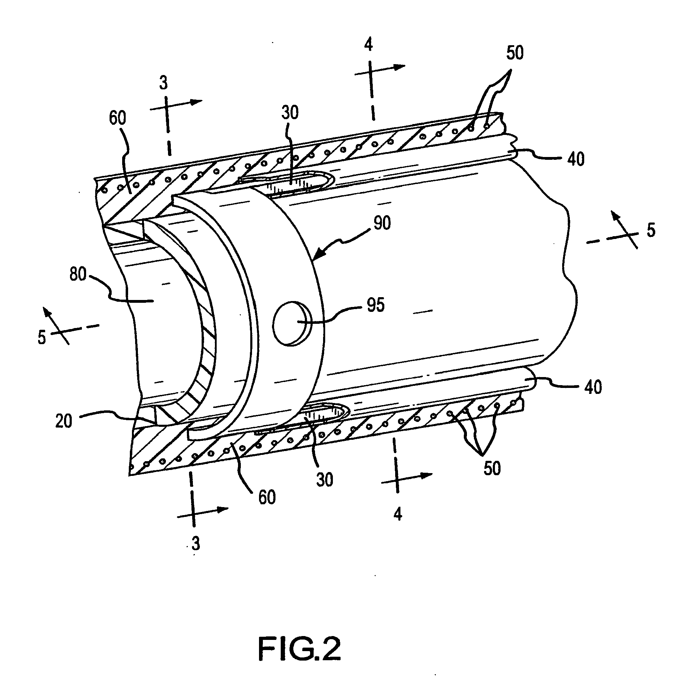

Figure 2 illustrates a perspective view of a catheter according to a preferred embodiment of the present invention, cut away to show details.

The basic method of manufacture of catheter 100 according to an embodiment of the present invention will be described with reference to Figures 2, 3, 4, 6, 7 and 8. As they are assembled, the catheter components will be collectively referred to as a catheter assembly.

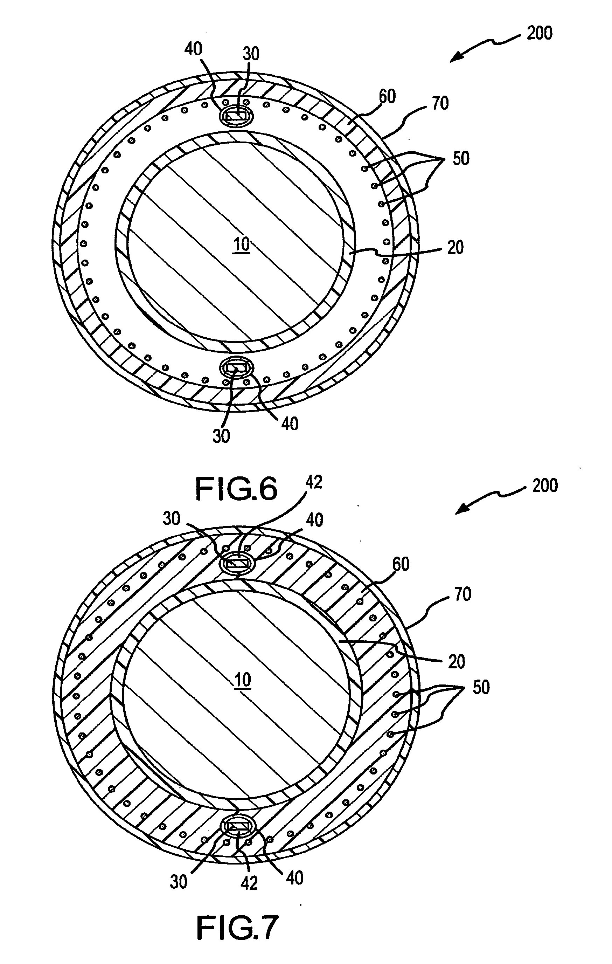

As depicted in Figure 6, a mandrel 10, which is preferably round in cross-section and preferably from about 152,4 mm 6 inches to about 1,2192 m 4 feet in length, is a component of the catheter assembly 200, and may be the first component thereof during manufacture of catheter 100. Mandrel 10 has a distal end and a proximal end. An inner liner 20 is placed on mandrel 10. Inner liner 20 may be knotted at one end (e.g. the distal end) and then fed onto mandrel 10.

Preferably, inner liner 20 is an extruded polytetrafluoroethylene (PTFE) tubing, such as Teflon® brand tubing, which is available commercially. Inner liner 20 may also be made of other melt processing polymers, including, without limitation, etched polytetrafluoroethylene, polyether block amides, nylon and other thermoplastic elastomers. Once such elastomer is Pebax®, made by Arkema, Inc. Pebax of various durometers may be used, including, without limitation, Pebax 30D to Pebax 70D. In a preferred embodiment, inner liner 20 is made of a material with a melting temperature higher than that of an outer layer 60, which will be further described below, such that inner liner 20 will withstand melt processing of outer layer 60.

A flat wire 30 is placed longitudinally along inner liner 20 Flat wire 30 is preferably composed of stainless steel and is preferably about 0,0508 mm 0.002" by about 0,1524 mm 0.006", and more preferably about 0,1016 0.004" by about 0,3048 mm 0.012". In one embodiment, at least a portion of flat wire 30 is encased inside another preformed tube 40 before placement along inner liner 20 to form a flat lumen 42. Preformed tube 40 need not have the same shape as the cross-section of flat wire 30, but instead may be round, oval, rectangular, or another like shape. Preferably, preformed tube 40 has a cross-section that is not the same shape as the cross-section of flat wire 30 in order to facilitate movement of flat wire 30 in preformed tube 40. Preformed tube 40 may be formed of polytetrafluoroethylene, polyether block amides, nylon, other thermoplastic elastomers, or another substance. Preferably, preformed tube 40 has a higher melting point than outer layer 60, which will be further described below, so that preformed tube 40 will not melt when outer layer 60 is subjected to melt processing.

In alternative embodiments, flat wire 30 may be covered with lubricious materials including silicone, Teflon®, siloxane, and other lubricious materials (not shown), before placement. Alternatively, flat wire 30 may also be coated with a lubricious layer to promote slideability. It is also contemplated that flat wire 30 may be manufactured witch a smooth surface to promote slideability. While stainless steel is a preferred material from which to compose flat wire 30, other materials may be used, including, without limitation, materials that are used for conventional round pull wires.

More than one flat wire 30 may also be used. In such cases, each such flat wire 30 may be encased inside its own flexible tube 40 to form separate flat lumens 42. Preferably, a pair of flat wires 30 are used, spaced apart about 180 degrees about the circumference of inner liner 20.

Outer layer 60 is then placed over inner liner 20, flat wires 30, and preformed tube 40 forming flat lumen 42. Outer layer 60 may be made of either single or multiple sections of tubing that may be either butted together or overlapped with each other. Preferably, outer layer 60 is an extruded polytetrafluoroethylene tubing, such as Teflon® brand tubing, which is available commercially. Outer layer 60 may also be made of other melt processing polymers, including, without limitation, etched polytetrafluoroethylene, polyether block amides, nylon and other thermoplastic elastomers. Once such elastomer is Pebax® made by Arkema, Inc. Pebax of various durometers may be used, including, without limitation, Pebax 30D to Pebax 70D. Outer layer 60 may also comprise more than one layer, including for example two or more tubes of a melt processing polymer.

Optionally, a braided wire assembly 50 may be placed over inner liner 20 and any flat wires 30 before outer layer 60 is applied. Braided wire assembly 50 may be formed of stainless steel wire, including for example 0,0762 mm 0.003" high tensile stainless steel wire. Braided wire assembly 50 may be formed in a standard braid pattern and density, for example, about 16 wires at about 45 to about 60 picks per inch ("PPI") density. Alternatively, a braid may be used that is characterized by a varying braid density. For example, braided wire assembly 50 may be characterized by a first braid density at proximal end 110 of catheter 100 and then transition to one or more different braid densities as braided wire assembly 50 approaches distal end 190 of catheter 100. The braid density of distal end 190 may be greater or less than the braid density at proximal end 110. In a specific example, the braid density at the base (i.e., proximal end 110) is about 50 PPI and the braid density at distal end 190 is about 10 PPI. In another embodiment, the braid density at distal end 190 is about 20% to about 35% of the braid density at the base/proximal end 110.

Braided wire assembly 50 may be formed separately on a disposable core. One or more portions of braided wire assembly 50 may be heat tempered and cooled before incorporation into catheter assembly 200 though methods that are known to those of ordinary skill. The action of heat tempering may help to release the stress on the wire and help reduce radial forces.

Figure 6 displays a cross-section of catheter assembly 200 having two flat wires 30 and braided wired assembly 50 encompassed by outer layer 60 before lamination of the materials by heating. In one preferred embodiment, a layer of heat shrink 70 is placed over the top of outer layer 60 as depicted in Figure 6. Heat shrink 70 is preferably a fluoropolymer or polyolefin material.

Figure 7 depicts catheter assembly 200 after a lamination process. Catheter assembly 200 may be laminated by heating catheter assembly 200 until the material comprising outer layer 60 flows and redistributes around the circumference thereof as depicted in Figure 7. Heat shrink 70 has a higher melting temperature than outer layer 60; and during the melt process, heat shrink 70 retains its tubular shape and forces the liquefied outer layer 60 material into braided wire assembly 50 (if present) and into contact with flat wires 30 and inner liner 20. Catheter assembly 200 may then be cooled. In Figure 7, mandrel 10 is still in place.

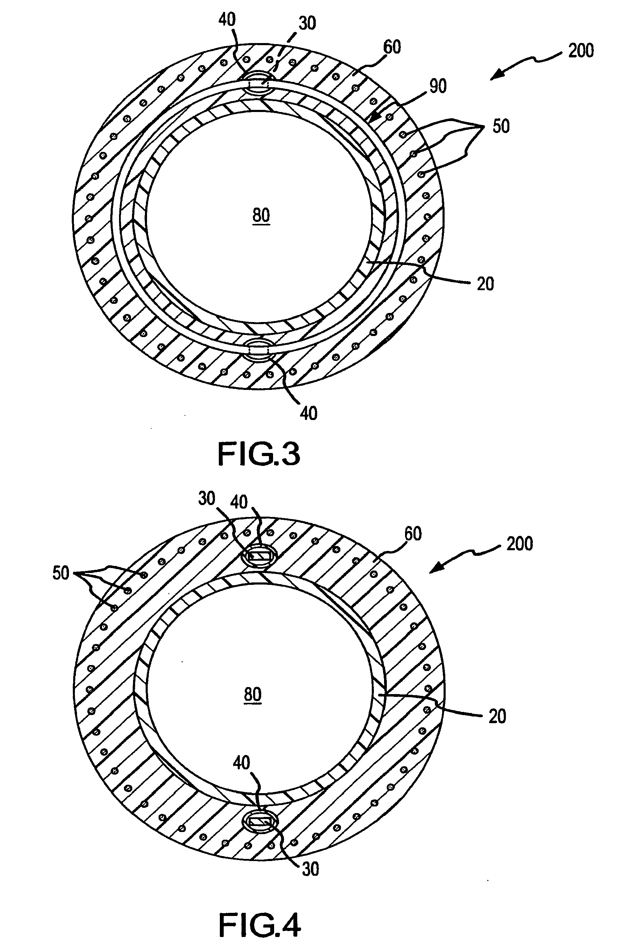

Mandrel 10 may be removed from catheter assembly 200, leaving behind a lumen 80 as illustrated in Figure 4, which depicts a catheter 100 made in accordance with the method of the present invention subsequent to the application of heat for the lamination process. Optionally, heat shrink 70 may be left in place around outer layer 60, as depicted in Figure 7, even after mandrel 10 is removed.

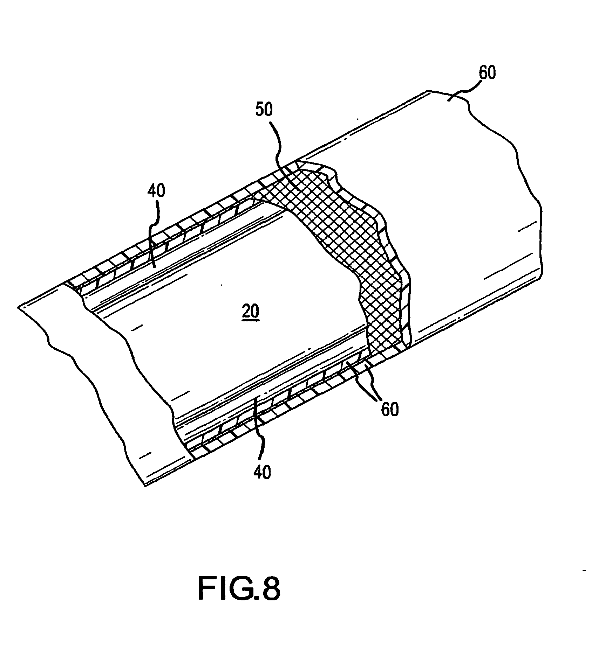

If heat shrink 70 is removed, outer layer 60 becomes the outermost layer of catheter 100. The result is a substantially circular catheter 100 with pull wires 30 embedded within outer layer 60 material as illustrated in Figures 3 and 4. Figure 3 is a cross-sectional view taken at the point of a pull ring 90 as depicted in Figure 2, while Figure 4 is a cross-sectional view taken at a point proximal to pull ring 90. Figure 8 is a perspective view of catheter assembly 200, cut away to show certain details of construction.

Catheter assembly 200 may be manufactured using alternative techniques. In one embodiment, outer layer 60 may be formed by extruding outer layer 60 over catheter assembly 200. In another embodiment, catheter assembly 200 may formed by using a combination of heat and a press that has a mold for defining the final shape of catheter 100.

Catheter 100 formed using the methods of this invention may have varying sizes and various uses. For example, catheter 100 may be used in atrial fibrillation cases as well as atrial tachycardia cases. In connection with certain heart applications, catheter 100 manufactured using the improvements discussed herein is preferably less than about 4 mm 12F outer diameter, and more preferably less than about 3,33 mm 10F outer diameter. For use as a steerable introducer, a catheter size of less than about 3,66 mm 11F outer diameter is preferred.

In another embodiment, catheter 100 construction may be modified to utilize materials of various durometer hardness (as measured, for example, using a Shore durometer hardness scale). For example, proximal end 110 of catheter 100 may be made of a material such as nylon 11, and the remainder of catheter 100 may be made of one or more Pebax materials. Preferably, the durometer hardness levels will decrease as catheter 100 shaft approaches distal end 190. For example, a nylon base may then be followed by one or more of the following Pebax segments: 70D Pebax; 60D Pebax; 55D Pebax; 40D Pebax; 35D Pebax; 30D Pebax. Catheter 100 may also use one or more blends of the foregoing Pebax materials, including for example, a 70D/60D Pebax blend made by co-extrusion, or a 40D/35D Pebax blend made by co-extrusion. Preferably, catheter 100 made with one or more segments of varying durometers will be reflowed together during manufacturing. The length of the segments may vary. Proximal end 110 of catheter 100 is preferably the longest segment, and more distal segments may preferably vary between about 6,35 mm 0.250" to about 152,4 mm 6", and more preferably from about 6,35 mm 0.25" to about 76,2 mm 3". Preferably, the hardness levels of the segments and the lengths of the segments may be adjusted for specific applications, and preferably, the distal tip segment may have the lowest durometer of all segments. The segments may be selected to optimize stability and torque delivery for the specific application.

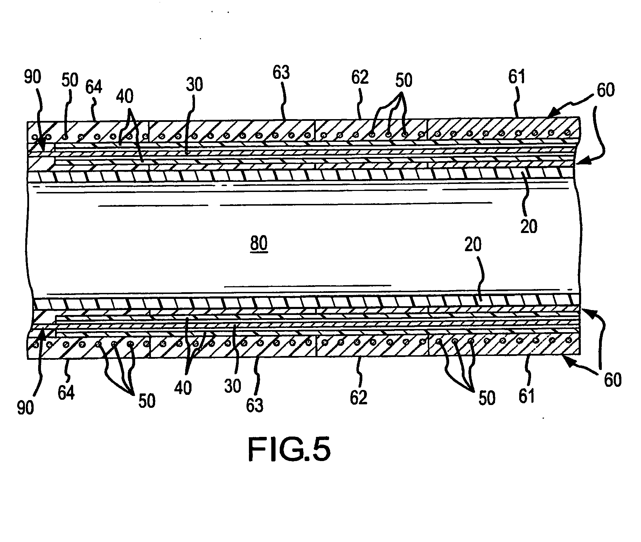

Figure 5 illustrates another embodiment of the invention in which outer layer 60 is composed of multiple segments 61, 62, 63, 64, each of which has different material properties, such as degree of hardness, stiffness, or tensile strength. In a preferred embodiment, segment 61 has the greatest degree of hardness; segments 62, 63, and 64 are more flexible than segment 61; segments 63 and 64 are more flexible than segments 61 and 62; and finally, segment 64 is more flexible than each of segments 61, 62 and 63. The number of segments may vary, as well as the relative lengths of the segments.

In yet another embodiment, a modified braided wire assembly 50 is inserted between inner liner 20 and outer layer 60. Braided wire assembly 50 may be designed to have transitional braid densities starting at one braid density and transitioning to a lower braid density. In one embodiment, the braid may begin at a braid density of about 50 to about 60 PPI, and more preferably between about 50 and about 55 PPI, and then transition to a braid density at the tip of about 5 to about 20 PPI, and more preferably between about 5 to about 15 PPI. The braid density may transition slowly, or it may change using one or more segments. For example, there may be an intermediate zone with a braid density of about 30 to about 45 PPI. Variations in the braid density of braided wire assembly 50 may be used to increase or decrease flexibility of catheter 100 depending on the desired application.

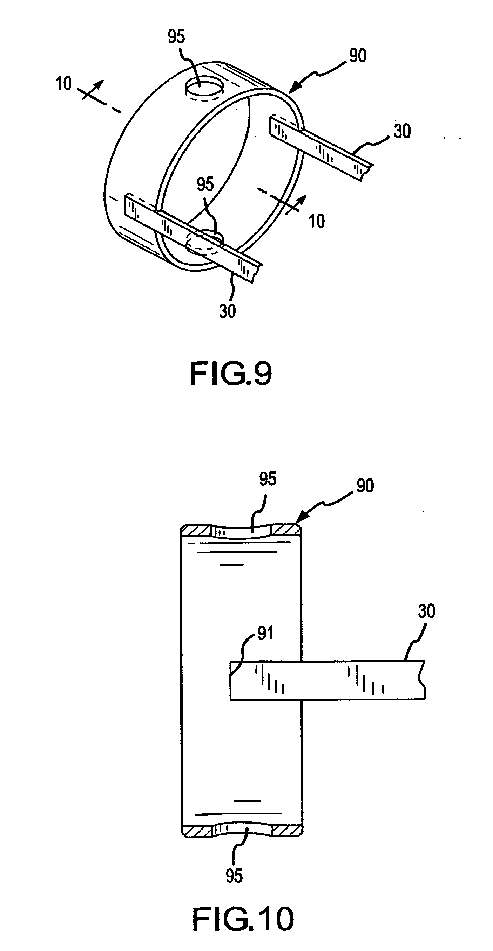

In another embodiment, pull ring 90 is utilized to provide steerability. Figures 9 and 10 illustrate a preferred embodiment for pull ring 90. Pull ring 90 is a generally circular band with a cross-sectional shape (measured orthogonally to a tangential line relative to the circle of the band) that is substantially rectangular. The rectangular cross-section is more clearly depicted in Figure 10. The outer dimension of pull ring 90 is preferably determined based on the application for catheter 100 to be manufactured. In one embodiment, pull ring 90 is about 0.10" in diameter.

Pull ring 90 preferably has at least one slot 91 that is configured to accommodate flat pull wire 30. Flat pull wire 30 may secured within slot 91 by any technique that is appropriate given the materials of pull ring 90 and flat pull wires 30. Acceptable techniques may include, but are not limited to, laser welding and/or other welding and bonding techniques.

In another embodiment, pull ring 90 may contain one or more flow holes 95 as illustrated in Figures 9 and 10. During a melting process, the material of outer layer 60 melts and flows through flow holes 95. Upon cooling, the material of outer layer 60 bonds to pull ring 90 to provide better adhesion between pull ring 90 and the remaining components of catheter assembly 200, thereby improving performance of catheter 100. While flow holes 95 are depicted as circular, other shapes may be used. In one embodiment, pull ring 90 includes two 0,635 mm 0.025" flow holes 95 spaced about 180 degrees apart around the circumference of pull ring 90. The size and shape of flow holes 95 may be adjusted based on the materials being used to form inner liner 20 and/or outer layer 60.

In another embodiment, pull ring 90 is utilized with non-flat pull wires. Pull ring 90 of this embodiment is preferably a circular band with a cross-sectional shape (measured orthogonally to a tangential line relative to the circle of the band) that is substantially rectangular. Preferably, pull ring 90 has at least one slot that is configured to accommodate a non-flat pull wire (such as a round wire). Preferably, the tip of the non-flat pull wire is tapered to facilitate joinder with pull ring 90. The non-flat pull wire may be secured within the slot by any technique that is appropriate given the materials of pull ring 90 and the pull wires. Acceptable techniques may include, but are not limited to, laser welding and/or other welding and bonding techniques. Preferably, the non-flat pull wire is located within a preformed tube. The preformed tube need not be the same shape as the cross-section of the pull wire, but instead, may be round, oval, rectangular, or another like shape. Preferably, the preformed tube has a cross-section that is not the same shape as the cross-section of the pull wire in order to facilitate movement of the pull wire in the preformed tube. The preformed tube may be formed of polytetrafluoroethylene, polyether bock amides, nylon, other thermoplastic elastomers or another substance. Preferably, the preformed tube has a higher melting point than outer layer 60 so that the preformed tube will not melt when outer layer 60 is subjected to melt processing. In alternative embodiments, the pull wire may be covered with lubricious materials, such as silicone and other lubricious materials, before placement. Alternatively, the pull wire may be coated with a lubricious layer to promote slideability, and it is also contemplated that the pull wire may be manufactured with a smooth surface to promote slideability. While stainless steel is a preferred material to compose the pull wire, other materials may be used, including, without limitation, materials that are used for conventional pull wires.

Pull ring 90 is typically utilized near distal end 190 of catheter 100, but it is anticipated that pull ring 90 may be located at any position along catheter 100. Moreover, more than one pull ring 90 may be utilized in the same catheter 100. In one embodiment of catheter 100, two separate pull rings 90 may be utilized, each of which has its own flat pull wires 30 connected thereto.

Although multiple embodiments of this invention have been described above with a certain degree of particularity, those skilled in the art could make numerous alterations to the disclosed embodiments without departing from the spirit or scope of this invention. For example, pull ring 90 may be made of stainless steel or other materials, including, without limitation, materials that are used to form conventional pull ring assemblies. In addition, braided wire assembly 50 may be made of stainless steel or other materials, including materials that are used to form conventional braided wire assemblies.

All directional references (e.g., upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use of the invention. Joinder references (e.g., attached, coupled, connected, secured and the like) are to be construed broadly and may include intermediate members between a connection of elements and relative movement between elements. As such, joinder references do not necessarily infer that two elements are directly connected and in fixed relation to each other.

It is intended that all matter contained in the above description or shown in the accompanying drawings shall be interpreted as illustrative only and not limiting. Changes in detail or structure may be made without departing from the spirit of the invention as defined in the appended claims.

## Claims

1. A catheter assembly (200), comprising:
an inner liner (20) made of flexible material; and
an outer layer (60) having a steering mechanism, the steering mechanism comprising at least one flat wire 30; and
a corresponding lumen (42) for each of the at least one flat wire (30) through which the flat wire (30) may travel.

2. The catheter assembly (200) of claim 1, further comprising a layer of heat shrink material (70) encompassing the outer layer (60), wherein the inner liner (20) icludes a central lumen (80), and wherein the catheter assembly (200) has a cross section with an outer shape that is substantially circular.

3. The catheter assembly (200) of claim 1 or 2, wherein the outer layer (60) comprises a melt processing polymer.

4. The catheter assembly (200) of claim 2 or 3, wherein the steering mechanism comprises at least one pull ring (90) to which the at least one flat wire (30) is secured, whereby the catheter assembly (200) may be steered by controlling the at least one flat wire (30).

5. The catheter assembly (200) of claim 4, wherein the pull ring (90) comprises at least two flow holes (95), said outer layer being bonded to the pull ring (90) such that the melt processing polymer occupies the at least two flow holes (95).

6. The catheter assembly (200) of claim 3, 4 or 5, wherein the steering mechanism comprises at least two flat wires (30) and at least two corresponding preformed tubes (40) through which the at least two flat wires (30) may travel, wherein the at least two flat wires (30) are secured to the at least one pull ring (90), and wherein the at least two preformed tubes (40) have cross-sections that are different in shape than a cross-section of the corresponding flat pull wires (30).

7. The catheter assembly (200) of claim 6, wherein the steering mechanism comprises a single pull ring (90) to which the at least two flat wires (30) are secured and the single pull ring (90) comprises a right circular cylinder having a slot for each of the at least two flat wires (30).

8. The catheter assembly (200) of claim 1, wherein the steering mechanism comprises at least two flat wires (30) and each of the at least two flat wires (30) has a cross-section that is rectangular, and wherein each of the at least two lumens (42) a cross-section selected from the group consisting of oval, round and elliptical.

9. The catheter assembly (200) of claim 1, wherein the steering mechanism comprises at least two flat wires (30) and each of the at least two flat wires (30) has a cross-section that is measured X in one direction and at least 3X in a second direction, said second direction being substantially orthogonal to the first direction.

10. The catheter assembly (200) of claim 1, wherein the steering mechanism comprises at least two flat wires (30) and wherein each of the at least two flat wires (30) is coated with a lubricious substance to permit the flat wire to slide in the corresponding lumen (42) or each of the at least two flat wires is manufactured with a smooth surface to reduce friction between the flat wire (30) and the corresponding lumen (42).

11. The catheter assembly (200) of claim 1, wherein the outer layer further (60) comprises a braided wire assemby (50).

12. The catheter assembly (200) of claim 11, wherein the braided wire assembly (50) extends from a base (110) of the catheter assembly (200) to a distal end (190) of the catheter assembly, and wherein the braided wire assembly (50) is **characterized by** a braid density that transitions from a first braid density at the base (110) to a lower braid density at the distal end (190).

13. The catheter assembly (200) of claim 12, wherein the braid density at the base (110) is about 50 PPI and the braid density at the distal end (190) is about 10 PPI.

14. The catheter assembly (200) of claim 12, where the braid density at the distal end (190) is about 20% to about 35% of the braid density at the base (110).

15. The catheter assembly (200) of claim 1, further comprising a catheter shaft with a distal portion and a proximal portion, said shaft being made of at least three segments, wherein each segment has a different hardness characteristic.

16. The catheter assembly (200) of claim 15, wherein the catheter shaft comprises:
a first segment (110) at the proximal portion of the catheter shaft, wherein the first segment comprises nylon;
a second segment (61) adjacent the first segment (110), said second segment (61) being closer to the distal portion than the first segment (110), wherein the second segment (61) comprises Pebax having a first durometer measurement; and
a third segment (62) adjacent the second segment (61), said third segment (62) being closer to the distal portion than the second (61) and first segments (110), wherein the third segment (62) comprises Pebax having a second durometer measurement, said second durometer measurement being a lower number on a durometer scale than said first durometer measurement.

17. The catheter assembly (200) of claim 15, wherein the catheter shaft comprises:
a first segment (110) at the proximal portion of the catheter shaft, wherein the first segment (110) comprises material having a first durometer measurement;
a second segment (61) adjacent the first segment (110), said second (61) segment being closer to the distal portion than the first segment (100), wherein the second segment (61) comprises material having a second durometer measurement, said second durometer measurement being a lower number on a durometer scale than said first durometer measurement; and
a third segment (62) adjacent the second segment (61), said third segment being closer to the distal portion than the second (61) and first segments (110), wherein the third segment (62) comprises material having a third durometer measurement, said third durometer measurement being a lower number on a durometer scale than said first and second durometer measurements.

18. The catheter assembly (200) of claim 15, wherein the catheter shaft comprises:
a first segment (110) at the proximal portion of the catheter shaft, wherein the first segment (110) comprises nylon;
a second segment (61) adjacent the first segment (110), said second (61) segment being closer to the distal portion than the first segment (110), wherein the second segment (61) comprises material having a first durometer measurement;
a third (62) segment adjacent the second (61) segment, said third (62) segment being closer to the distal portion than the second segment (61), wherein the second segment (61) comprises material having a second durometer measurement, said second durometer measurement being a lower number on a durometer scale than said first durometer measurement;
a fourth segment (63) adjacent the third segment (62), said fourth segment (63) being closer to the distal portion than the third segment (62), wherein the second segment (61) comprises material having a third durometer measurement, said third durometer measurement being a lower number on a durometer scale than said second durometer measurement; and
a fifth (64) segment adjacent the fourth segment (63), said fifth segment (64) being closer to the distal portion than the fourth segment (63), wherein the fourth segment (63) comprises material having a fourth durometer measurement, said fourth durometer measurement being a lower number on a durometer scale than said third durometer measurement.

19. A method of manufacturing a steerable introducer catheter, comprising the steps of:
providing a mandrel (10);
laminating the mandrel (10) with a lining material to form an inner liner (20);
providing at least one flat shaped wire (30);
covering the inner liner (20) and the at least one flat shaped wire (30) with a melt processing polymer;
applying sufficient heat to the melt processing polymer to raise the temperature of the melt processing polymer above its melting point;
cooling the assembly; and
removing the mandrel (10), thereby forming a steerable introducer catheter.

20. The method of claim 19, further comprising:
placing a flexible tube over each of the at least one flat shaped wires (30) to create at least one corresponding lumen (42) for each of the at least one flat shaped wire (30).

21. The method of claim 20, wherein the step of providing at least one flat shaped wire (30) comprises providing at least one flat wire (30) having a cross-section that is rectangular, and wherein the preformed flexible tube has a cross-section selected from the group consisting of oval, round, and elliptical.

22. The method of claims 19-21, further comprising:
covering the melt processing polymer with shrink wrap tubing(70); and
removing the shrink wrap tubing (70) after the melting process.

23. The method of claims 19 - 22, further comprising:
placing a braided wire assembly (50) over the inner liner (20) and the at least one flat lumen (42).

24. The method of claim 23, further comprising:
covering the braided wire assembly (50) with one or more flexible layers.

25. The method of claims 19-24, wherein the melt processing polymer is selected from the group consisting of Nylon and Pebax.

26. The method of claim 20, wherein the step of placing a flexible liner over each of the at least one flat shaped wires further comprises placing the flexible tube over the at least one flat lumen and the inner liner.

27. The method of claims 19-26, wherein the material comprising the inner liner is PTFE.

28. The method of claims 19-27, wherein the catheter being manufactured has an outer diameter that is less than about 4 mm 12F.

## Patentansprüche

1. Katheteranordnung (200) mit:
einem aus einem biegsamen Material bestehenden Innenmantel (20); und
einer einen Lenkmechanismus umfassenden Außenschicht (60), wobei der Lenkmechanismus umfasst:
wenigstens einen flachen Draht (30); und
ein entsprechendes Lumen (42) für jeden der wenigstens einen flachen Drähte (30), durch das der flache Draht (30) verlaufen kann.

2. Katheteranordnung (200) gemäß Anspruch 1, weiterhin mit einer Schicht von Wärmeschrumpfmaterial (70), das die Außenschicht (60) umschließt, wobei der Innenmantel (20) ein zentrales Lumen (80) aufweist und wobei die Katheteranordnung (200) einen Querschnitt aufweist, der eine äußere Form besitzt, die im Wesentlichen kreisförmig ist.

3. Katheteranordnung (200) gemäß Anspruch 1 oder 2, wobei die Außenschicht (60) ein thermoplastisches Polymer aufweist.

4. Katheteranordnung (200) gemäß Anspruch 2 oder 3, wobei der Lenkmechanismus wenigstens einen Zugring (90) aufweist, an dem der wenigstens eine flache Draht (30) befestigt ist, wodurch die Katheteranordnung (200) durch Steuern des wenigstens einen flachen Drahtes (30) gesteuert werden kann.

5. Katheteranordnung (200) gemäß Anspruch 4, wobei der Ziehring (90) wenigstens zwei Strömungsbohrungen (95) aufweist, wobei die Außenschicht (60) an dem Ziehring (90) gebunden ist, dass das thermoplastische Polymer die wenigstens zwei Strömungslöcher (95) ausfüllt.

6. Katheteranordnung (200) gemäß Anspruch 3, 4 oder 5, wobei der Lenkmechanismus wenigstens zwei flache Drähte (30) und wenigstens zwei entsprechend vorgeformte Schläuche (40) aufweist, durch die die wenigstens zwei flachen Drähte (30) verlaufen können, wobei die wenigstens zwei flachen Drähte (30) an dem wenigstens einen Ziehring (90) befestigt sind und wobei die wenigstens zwei vorgeformten Schläuche (40) Querschnitte aufweisen, die eine sich von einem Querschnitt des entsprechenden flachen Drahtes (30) unterscheidende Form besitzen.

7. Katheteranordnung (200) gemäß Anspruch 6, wobei der Lenkmechanismus einen einzelnen Ziehring (90) umfasst, an dem die wenigstens zwei flachen Drähte (30) befestigt sind und wobei der einzelne Ziehring (90) einen geraden Kreiszylinder aufweist, der eine Aufnahme für jeden der wenigstens zwei flachen Drähte (30) besitzt.

8. Katheteranordnung (200) gemäß Anspruch 1, wobei der Lenkmechanismus wenigstens zwei flache Drähte (30) umfasst und wobei jeder der wenigstens zwei flachen Drähte (30) einen rechtwinkligen Querschnitt aufweist und wobei jeder der wenigstens zwei Lumen (42) einen Querschnitt aufweist, der aus der Gruppe bestehend aus oval, rund und elliptisch ausgewählt ist.

9. Katheteranordnung (200) gemäß Anspruch 1, wobei der Lenkmechanismus wenigstens zwei flache Drähte (30) umfasst und wobei die wenigstens zwei flachen Drähte (30) einen Querschnitt haben, der in einer Richtung X misst und wenigstens 3X in einer zweiten Richtung misst, wobei die zweite Richtung im Wesentlichen orthogonal zu der ersten Richtung ist.

10. Katheteranordnung (200) gemäß Anspruch 1, wobei der Lenkmechanismus zumindest zwei flache Drähte (30) umfasst und wobei jeder der wenigstens zwei flachen Drähte (30) mit einer gleitfähigen Substanz beschichtet ist, um es dem flachen Draht zu ermöglichen, in dem entsprechenden Lumen (42) zu gleiten oder jeder der wenigstens zwei flachen Drähte (30) mit einer glatten Oberfläche gefertigt ist, um eine Reibung zwischen dem flachen Draht (30) und dem entsprechenden Lumen (42) zu reduzieren.

11. Katheteranordnung (200) gemäß Anspruch 1, wobei die Außenschicht (60) weiterhin eine geflochtene Drahtanordnung (50) umfasst.

12. Katheteranordnung (200) gemäß Anspruch 11, wobei sich die geflochtene Drahtanordnung (50) von einer Basis (110) der Katheteranordnung (200) zu einem distalen Ende (190) der Katheteranordnung erstreckt, und wobei die geflochtene Drahtanordnung (50) durch eine Geflechtdicke gekennzeichnet ist, die von einer ersten Geflechtdicke bei der Basis (110) zu einer niedrigeren Geflechtdicke bei dem distalen Ende (190) übergeht.

13. Katheteranordnung (200) gemäß Anspruch 12, wobei die Geflechtdichte bei der Basis (110) circa 50 Punkte pro Zoll und die Geflechtdichte bei dem distalen Ende (190) circa 10 Punkte pro Zoll beträgt.

14. Katheteranordnung (200) gemäß Anspruch 12, wobei die Geflechtdichte bei dem distalen Ende (190) circa 20% bis circa 35% der Geflechtdichte bei der Basis (110) beträgt.

15. Katheteranordnung (200) gemäß Anspruch 1, weiterhin mit einem Katheterschaft mit einem distalen Bereich und einem proximalen Bereich, wobei der Schaft aus wenigstens drei Bereichen besteht, wobei jeder Bereich eine unterschiedliche Härteeigenschaft aufweist.

16. Katheteranordnung (200) gemäß Anspruch 15, wobei der Katheterschaft umfasst:
einen ersten Abschnitt (110) bei dem proximalen Bereich des Katheterschafts, wobei der erste Abschnitt Nylon aufweist;
einem an den ersten Abschnitt (110) angrenzenden zweiten Abschnitt (61), wobei der zweite Abschnitt (61) näher am distalen Bereich als der erste Abschnitt (110) ist, wobei der zweite Abschnitt (61) Pebax aufweist, das einen ersten Härtewert besitzt; und
einem an den zweiten Abschnitt (61) angrenzenden dritten Abschnitt (62), wobei der dritte Abschnitt (62) näher am distalen Bereich als der zweite Abschnitt (61) und der erste Abschnitt (110) ist, wobei der dritte Abschnitt (62) Pebax aufweist, das einen zweiten Härtewert besitzt, wobei der zweite Härtewert ein niedrigerer Wert auf einer Härteskala als der erste Härtewert ist.

17. Katheteranordnung (200) gemäß Anspruch 15, wobei der Katheterschaft umfasst:
einen ersten Abschnitt (110) bei dem proximalen Bereich des Katheterschafts, wobei der erste Abschnitt (110) Material aufweist, das einen ersten Härtewert besitzt;
einen an den ersten Abschnitt (110) angrenzenden zweiten Abschnitt (61), wobei der zweite Abschnitt (61) näher am distalen Bereich als der erste Abschnitt (110) ist, wobei der zweite Abschnitt (61) Material aufweist, das einen zweiten Härtewert besitzt, wobei der zweite Härtewert ein niedrigerer Wert auf einer Härteskala als der erste Härtewert ist; und
einen an den zweiten Abschnitt (61) angrenzenden dritten Abschnitt (62), wobei der dritte Abschnitt näher am distalen Bereich als der zweite (61) und erste Abschnitt (110) ist, wobei der dritte Abschnitt (62) ein Material aufweist, das einen dritten Härtewert besitzt, wobei der dritte Härtewert ein niedrigerer Wert auf einer Härteskala als der erste und zweite Härtewert ist.

18. Katheteranordnung (200) gemäß Anspruch 15, wobei der Katheterschaft umfasst:
einen ersten Abschnitt (110) bei einem proximalen Bereich des Katheterschafts, wobei der erste Abschnitt (110) Nylon aufweist;
einen an den ersten Abschnitt (110) angrenzenden zweiten Abschnitt (61), wobei der zweite Abschnitt (61) näher am distalen Bereich als der erste Abschnitt (110) ist, wobei der zweite Abschnitt (61) Material aufweist, das einen ersten Härtewert besitzt;
einen an den zweiten Abschnitt (61) angrenzenden dritten Abschnitt (62), wobei der dritte Abschnitt (62) näher an dem distalen Bereich als der zweite Abschnitt (61) ist, wobei der zweite Abschnitt (61) Material aufweist, das einen zweiten Härtewert besitzt, wobei der zweite Härtewert ein niedrigerer Wert auf einer Härteskala als der erste Härtewert ist;
einen an den dritten Abschnitt (62) angrenzenden vierten Abschnitt (63), wobei der vierte Abschnitt (63) näher an dem distalen Bereich als der dritte Abschnitt (62) ist, wobei der zweite Bereich (61) ein Material aufweist, das einen dritten Härtewert besitzt, wobei der dritte Härtewert ein niedrigerer Wert auf einer Härteskala als der zweite Härtewert ist; und
einen an den vierten Abschnitt (63) angrenzenden fünften Abschnitt (64), wobei der fünfte Abschnitt (64) näher an dem distalen Bereich als der vierte Abschnitt (63) ist, wobei der vierte Abschnitt (63) ein Material aufweist, das einen vierten Härtewert besitzt, wobei der vierte Härtewert ein niedrigerer Wert auf einer Härteskala als der dritte Härtewert ist.

19. Verfahren zum Herstellen eines lenkbaren Einführkatheters mit den Schritten:
Bereitstellen eines Doms (10);
Beschichten des Doms mit einem Auskleidungsmaterial zum Formen des Innenmantels (20);
Bereitstellen wenigstens eines flachen Drahtes (30);
Überziehen des Innenmantels (20) und des wenigstens einen flachen Drahtes (30) mit einem thermoplastischen Polymer;
Aufbringen von ausreichender Hitze auf das thermoplastische Polymer zum Erhöhen der Temperatur des thermoplastischen Polymers oberhalb seines Schmelzpunkts;
Kühlen der Anordnung; und
Entfernen des Doms (10), wodurch ein lenkbarer Einführkatheter gebildet wird.

20. Verfahren gemäß Anspruch 19 weiterhin mit:
Vorsehen eines biegsamen Schlauchs über jeden der wenigstens einen flach geformten Drähte(30) zum Erzeugen von wenigstens einem entsprechenden Lumen (42) für jeden der wenigstens einen flach geformten Drähte (30).

21. Verfahren gemäß Anspruch 20, wobei der Schritt des Bereitstellens von wenigstens einem flach geformten Draht (30) ein Bereitstellen von wenigstens einem einen rechteckigen Querschnitt aufweisenden flachen Draht (30) umfasst, und wobei der vorgeformte biegsame Schlauch einen Querschnitt aufweist, der aus der Gruppe bestehend aus oval, rund und elliptisch ausgewählt ist.

22. Verfahren gemäß Anspruch 19 bis 21 weiterhin mit:
Überziehen des thermoplastischen Polymers mit einem Schrumpffolienschlauch (70); und
Entfernen des Schrumpffolienschlauchs (70) nach dem Schmelzprozess.

23. Verfahren gemäß Ansprüchen 19 bis 22 weiterhin mit:
Vorsehen einer geflochtenen Drahtanordnung (50) über dem Innenmantel (20) und dem wenigstens einen flachen Lumen (42).

24. Verfahren gemäß Anspruch 23 weiterhin mit:
Überziehen der geflochtenen Drahtanordnung (50) mit einer oder mehreren biegsamen Schichten.

25. Verfahren gemäß Anspruch 19 bis 24, wobei das thermoplastische Polymer aus der Gruppe bestehend aus Nylon und Pebax ausgewählt ist.

26. Verfahren nach Anspruch 20, wobei der Schritt des Vorsehens eines biegsamen Mantels über jeden der wenigstens einen flach geformten Drähte weiterhin ein Vorsehen des biegsamen Schlauchs über das wenigstens eine flache Lumen und des Innenmantels umfasst.

27. Verfahren gemäß Ansprüchen 19 bis 26, wobei das Material des Innenmantels PTFE aufweist.

28. Verfahren gemäß den Ansprüchen 19 bis 27, wobei der hergestellte Katheter einen Außendurchmesser aufweist, der kleiner als ca. 4 mm (12 F) beträgt.

## Revendications

1. Ensemble formant cathéter (200), comprenant :
un habillage intérieur (20) fabriqué à partir d'un matériau flexible ; et
une couche externe (60) dotée d'un mécanisme de direction, le mécanisme de direction comprenant :
au moins un fil plat (30) ; et
une lumière (42) correspondante pour chacun de l'au moins un fil plat (30) à travers laquelle le fil plat (30) peut passer.

2. Ensemble formant cathéter (200) selon la revendication 1, comprenant en outre une couche de matériau thermorétractable (70) englobant la couche externe (60), dans lequel l'habillage intérieur (20) comprend une lumière centrale (80), et dans lequel l'ensemble formant cathéter (200) possède une section transversale présentant une forme externe qui est sensiblement circulaire.

3. Ensemble formant cathéter (200) selon la revendication 1 ou 2, dans lequel la couche externe (60) comprend un polymère obtenu par traitement à l'état fondu.

4. Ensemble formant cathéter (200) selon la revendication 2 ou 3, dans lequel le mécanisme de direction comprend au moins un anneau d'arrachage (90) sur lequel l'au moins un fil plat (30) est fixé, moyennant quoi l'ensemble formant cathéter (200) peut être orienté en contrôlant l'au moins un fil plat (30).

5. Ensemble formant cathéter (200) selon la revendication 4, dans lequel l'anneau d'arrachage (90) comprend au moins deux orifices de passage (95), ladite couche externe (60) étant reliée à l'anneau d'arrachage (90) de manière à ce que le polymère obtenu par traitement à l'état fondu occupe les au moins deux orifices de passage (95).

6. Ensemble formant cathéter (200) selon la revendication 3, 4 ou 5, dans lequel le mécanisme de direction comprend au moins deux fils plats (30) et au moins deux tubes préformés (40) correspondants à travers lesquels les au moins deux fils plats (30) peuvent passer, dans lequel les au moins deux fils plats (30) sont fixés à l'au moins un anneau d'arrachage (90), et dans lequel les au moins deux tubes préformés (40) possèdent une section transversale dont la forme est différente de celle d'une section transversale des fils de traction plats (30) correspondants.

7. Ensemble formant cathéter (200) selon la revendication 6, dans lequel le mécanisme de direction comprend un anneau d'arrachage (90) unique sur lequel les au moins deux fils plats (30) sont fixés et l'anneau d'arrachage (90) unique comprend un cylindre circulaire droit doté d'une encoche pour chacun des au moins deux fils plats (30).

8. Ensemble formant cathéter (200) selon la revendication 1, dans lequel le mécanisme de direction comprend au moins deux fils plats (30) et chacun des au moins deux fils plats (30) possède une section transversale qui est rectangulaire, et dans lequel chacune des au moins deux lumières (42) possède une section transversale choisie dans le groupe constitué des formes ovale, ronde et elliptique.

9. Ensemble formant cathéter (200) selon la revendication 1, dans lequel le mécanisme de direction comprend au moins deux fils plats (30) et chacun des au moins deux fils plats (30) possède une section transversale dont la mesure est X suivant une direction et au moins 3X suivant une deuxième direction, ladite deuxième direction étant sensiblement orthogonale à la première direction.

10. Ensemble formant cathéter (200) selon la revendication 1, dans lequel le mécanisme de direction comprend au moins deux fils plats (30) et chacun des au moins deux fils plats (30) est revêtu d'une substance lubrifiante pour permettre au fil plat de glisser dans la lumière (42) correspondante ou chacun des au moins deux fils plats (30) présente une surface lisse pour réduire la friction entre le fil plat (30) et la lumière (42) correspondante.

11. Ensemble formant cathéter (200) selon la revendication 1, dans lequel la couche externe (60) comprend en outre un ensemble de fils tressés (50).

12. Ensemble formant cathéter (200) selon la revendication 11, dans lequel l'ensemble de fils tressés (50) s'étend depuis une base (110) de l'ensemble formant cathéter (200) jusqu'une extrémité distale (190) de l'ensemble formant cathéter, et dans lequel l'ensemble de fils tressés (50) est **caractérisé par** une densité de tresses qui passe d'une première densité de tresses à la base (110) à une densité de tresses inférieure au niveau de l'extrémité distale (190).

13. Ensemble formant cathéter (200) selon la revendication 12, dans lequel la densité de tresses à la base (110) est d'environ 50 duites au pouce et la densité de tresses au niveau de l'extrémité distale (190) est d'environ 10 duites au pouce.

14. Ensemble formant cathéter (200) selon la revendication 12, dans lequel la densité de tresses au niveau de l'extrémité distale (190) est d'environ 20 % à environ 35 % de la densité de tresses à la base (110).

15. Ensemble formant cathéter (200) selon la revendication 1, comprenant en outre une tige de cathéter présentant une partie distale et une partie proximale, ladite tige étant composée d'au moins trois segments, chaque segment présentant une dureté différente.

16. Ensemble formant cathéter (200) selon la revendication 15, dans lequel la tige de cathéter comprend :
un premier segment (110) au niveau de la partie proximale de la tige de cathéter, le premier segment comprenant du nylon ;
un deuxième segment (61) adjacent au premier segment (110), ledit deuxième segment (61) étant plus proche de la partie distale que le premier segment (110), le deuxième segment (61) comprenant du Pebax ayant une première mesure de dureté au duromètre ; et
un troisième segment (62) adjacent au deuxième segment (61), ledit troisième segment (62) étant plus proche de la partie distale que le deuxième (61) et le premier (110) segments, le troisième segment (62) comprenant du Pebax ayant une deuxième mesure de dureté au duromètre, ladite deuxième mesure de dureté au duromètre étant un nombre inférieur sur une échelle de duromètre à ladite première mesure de dureté au duromètre.

17. Ensemble formant cathéter (200) selon la revendication 15, dans lequel la tige de cathéter comprend :
un premier segment (110) au niveau de la partie proximale de la tige de cathéter, le premier segment (110) comprenant un matériau ayant une première mesure de dureté au duromètre ;
un deuxième segment (61) adjacent au premier segment (110), ledit deuxième segment (61) étant plus proche de la partie distale que le premier segment (110), le deuxième segment (61) comprenant un matériau ayant une deuxième mesure de dureté au duromètre, ladite deuxième mesure de dureté au duromètre étant un nombre inférieur sur une échelle de duromètre à ladite première mesure de dureté au duromètre ; et
un troisième segment (62) adjacent au deuxième segment (61), ledit troisième segment étant plus proche de la partie distale que le deuxième (61) et le premier (110) segments, le troisième segment (62) comprenant un matériau ayant une troisième mesure de dureté au duromètre, ladite troisième mesure de dureté au duromètre étant un nombre inférieur sur une échelle de duromètre auxdites première et deuxième mesures de dureté au duromètre.

18. Ensemble formant cathéter (200) selon la revendication 15, dans lequel la tige de cathéter comprend :
un premier segment (110) au niveau de la partie proximale de la tige de cathéter, le premier segment (110) comprenant du nylon ;
un deuxième segment (61) adjacent au premier segment (110), ledit deuxième segment (61) étant plus proche de la partie distale que le premier segment (110), le deuxième segment (61) comprenant un matériau ayant une première mesure de dureté au duromètre ;
un troisième segment (62) adjacent au deuxième segment (61), ledit troisième segment (62) étant plus proche de la partie distale que le deuxième segment (61), le deuxième segment (61) comprenant un matériau ayant une deuxième mesure de dureté au duromètre, ladite deuxième mesure de dureté au duromètre étant un nombre inférieur sur une échelle de duromètre à ladite première mesure de dureté au duromètre ;
un quatrième segment (63) adjacent au troisième segment (62), ledit quatrième segment (63) étant plus proche de la partie distale que le troisième segment (62), le deuxième segment (61) comprenant un matériau ayant une troisième mesure de dureté au duromètre, ladite troisième mesure de dureté au duromètre étant un nombre inférieur sur une échelle de duromètre à ladite deuxième mesure de dureté au duromètre ; et
un cinquième segment (64) adjacent au quatrième segment (63), ledit cinquième segment (64) étant plus proche de la partie distale que le quatrième segment (63), le quatrième segment (63) comprenant un matériau ayant une quatrième mesure de dureté au duromètre, ladite quatrième mesure de dureté au duromètre étant un nombre inférieur sur une échelle de duromètre à ladite troisième mesure de dureté au duromètre.

19. Procédé de fabrication d'un cathéter à dispositif d'introduction orientable, comprenant les étapes consistant à :
prévoir un mandrin (10) ;
laminer le mandrin (10) avec un matériau d'habillage pour former un habillage intérieur (20) ;
prévoir au moins un fil plat (30) ;
recouvrir l'habillage intérieur (20) et l'au moins un fil plat (30) d'un polymère obtenu par traitement à l'état fondu ;
appliquer suffisamment de chaleur sur le polymère obtenu par traitement à l'état fondu pour élever la température du polymère obtenu par traitement à l'état fondu au-dessus de son point de fusion ;
refroidir l'ensemble ; et
retirer le mandrin (10), en formant ainsi un cathéter à dispositif d'introduction orientable.

20. Procédé selon la revendication 19, comprenant en outre :
la mise en place d'un tube flexible sur chacun de l'au moins un fil plat (30) pour créer au moins une lumière (42) correspondante pour chacun de l'au moins un fil plat (30).

21. Procédé selon la revendication 20, dans lequel l'étape de fourniture d'au moins un fil plat (30) comprend la fourniture d'au moins un fil plat (30) possédant une section transversale rectangulaire, et dans lequel le tube flexible préformé possède une section transversale choisie dans le groupe constitué des formes ovale, ronde et elliptique.

22. Procédé selon les revendications 19 à 24, comprenant en outre :
le revêtement du polymère obtenu par traitement à l'état fondu avec un tube enveloppant rétractable (70) ; et
le retrait du tube enveloppant rétractable (70) après le procédé de fusion.

23. Procédé selon les revendications 19 à 22, comprenant en outre :
la mise en place d'un ensemble de fils tressés (50) sur l'habillage intérieur (20) et l'au moins une lumière plate (42).

24. Procédé selon la revendication 23, comprenant en outre :
le revêtement de l'ensemble de fils tressés (50) avec une ou plusieurs couches flexibles.

25. Procédé selon les revendications 19 à 24, dans lequel le polymère obtenu par traitement à l'état fondu est choisi dans le groupe constitué du Nylon et du Pebax.

26. Procédé selon la revendication 20, dans lequel l'étape de mise en place d'un habillage flexible sur chacun de l'au moins un fil plat comprend en outre la mise en place du tube flexible sur l'au moins une lumière plate et l'habillage intérieur.

27. Procédé selon les revendications 19 à 26, dans lequel le matériau comprenant l'habillage intérieur est du PTFE.

28. Procédé selon les revendications 19 à 27, dans lequel le cathéter fabriqué a un diamètre externe qui est inférieur à environ 4 mm (12F).
